# EUROPEAN PATENT SPECIFICATION

(11) **EP 3 492 122 B1**
(45) Date of publication and mention of the grant of the patent: **01.07.2020**
(21) Application number: 19152867.8
(22) Date of filing: 19.04.2012
(51) Int. Cl.: A61M 5/142, A61M 1/10, F04B 43/12, F04B 43/00, F16L 11/12

(54) **EXTRA-CORPOREAL CIRCUIT**
EXTRAKORPORALER KREISLAUF
CIRCUIT EXTRACORPOREL

(30) Priority: 21.04.2011 EP 11425110
(43) Date of publication of application: 05.06.2019
(62) Divisional of application: 12715389.8
(73) Proprietor: SIS-TER S.p.A., 26020 Palazzo Pignano (Cremona) (IT)
(72) Inventor: FINI, Massimo, 41037 Mirandola (MO) (IT); REITER, Reinhold, 26013 Crema (CR) (IT)
(74) Representative: Boggio Merlo, Anita

(56) References cited:
- WO-A1-00/70225
- GB-A- 1 378 361
- GB-A- 1 380 812
- US-A- 3 737 256
- US-A- 4 558 996
- US-A- 5 468 129
- US-A1- 2010 329 910

## Description

The invention relates to a circuit for extra-corporeal circulation and in particular to the tubular insert intended to co-operate with a peristaltic pump.

In therapeutic treatments which require the use of extra-corporeal circulation, for example during haemodialysis treatments, it is required to ensure circulation of the blood and/or of the other physiological fluids along the pipes forming the circuit.

For this purpose it is known to use peristaltic pumps comprising a stator and a rotor between which a flexible tube is inserted. The rotor of the pump comprises rollers, usually two rollers, suitable for pressing the tube against the stator. The combined action of the pressure exerted by the rollers and the rotation imparted by the rotor causes displacement of the liquid between the two rollers inside the tube. The subsequent and constant displacement of portions of liquid produces, in a known manner, pumping of the liquid along the circuit.

The extra-corporeal circulation normally lasts, in the case of an ordinary haemodialysis treatment, for about 4 hours. However, cases may arise - for example, those in which it is required to treat an acute renal insufficiency or even more complex clinical conditions affecting the functioning of several organs - where the extra-corporeal circulation must be maintained for very long periods of time, for example 24 or even 48 hours.

As the person skilled in the art can easily understand, the correct functioning of the peristaltic pump and the entire circuit, and therefore the success of the therapeutic treatment as a whole, depend to a large extent on the characteristics of the tube portion co-operating with the peristaltic pump itself.

First and foremost, the tube must be able to satisfy two requirements which are substantially of an opposing nature. On the one hand, the walls of the tube must be sufficiently soft to allow proper compression by the rotor rollers. The proper compression of the tube, resulting in the complete occlusion of its inner lumen, allows an efficient suction phase. On the other hand, the tube walls must be sufficiently rigid to allow a good elastic recovery at the end of the compression exerted by the rollers. Compression of the tube and subsequent elastic recovery thereof constitute in fact the basis for correct operation of the peristaltic pump in terms of volumetric flow rate. It is also to be considered that operation of the circuit may in some cases start with a cold liquid which lowers the temperature of the tube itself. As it is well known, low temperature conditions negatively affect the characteristics of the polymer which forms the tubular insert.

Secondly, the tube must be able to ensure that, during the period of time for which it is used, it does not give rise to a deterioration in the performance, typically in terms of the volumetric flow rate of the peristaltic pump.

In view of the above comments, the tubes which are intended to co-operate with peristaltic pumps usually undergo tests which envisage the worst possible scenario, i.e. continuous operation for 24 to 48 hours.

Until recently, the tubes intended to co-operate with peristaltic pumps were made of PVC (polyvinyl chloride) plasticized with DOP (dioctyl phthalate, also called diisooctyl phthalate, di-2-ethyl hexyl phthalate or DEHP). This material (referred to below as PVC-DOP) ensured acceptable performance for the new, unused tube as well as a limited deterioration in performance in the case of prolonged use. Expressed in numerical terms, it is considered that the unused tubes made of PVC-DOP with an internal diameter of 8 mm ensured an average flow rate of about 320 ml/min and were subject, after 6 hours' uninterrupted operation, to a reduction in flow rate of between 8% and 10%.

Recently, following specific toxicological studies, standards have been introduced with the aim of eliminating DOP from products intended for use in a medical environment and in particular products which are exposed to systematic and prolonged contact with patients' blood.

In view of the above, recently products intended for medical use and made of materials other than PVC-DOP have been proposed. In particular, in connection with circuits used for haemodialysis, it has been proposed to replace PVC-DOP with silicone. Silicone ensures that optimum characteristics are obtained and that these are also maintained during long periods of operational use. Silicone, however, is much more costly than PVC and its widespread use would result in an increase in costs which would be unacceptable for most health systems.

In addition it has been proposed using PVC and replacing only the DOP plasticizer with another known plasticizer such as TOTM (trioctyl trimellitate). This solution, although decidedly less expensive than silicone, results, however, in a slight increase in costs compared to the conventional solution PVC-DOP solution. The main problem with PVC-TOTM consists, however, in the fact that this material does not ensure a sufficient elasticity. In other words, a tube made of PVC-TOTM and intended for use in a peristaltic pump is hardly able to satisfy both the requirements which, as mentioned above, involve characteristics of an opposing nature. More particularly, a tube made of PVC-TOTM, having a low hardness suitable to be effectively operated in suction conditions even with cold liquids, presents an unacceptable deterioration in its performance with prolonged use and even in a relatively short amount of time comparable to a standard 4-hour haemodialysis treatment. The choice of increasing the wall thickness of the PVC-TOTM tube so as to ensure an effective elastic recovery is not feasible because of the existence of standards about connectors and other components. Moreover, if the thickness of the wall was increased by reducing the inner diameter of the tube, considerable alterations would arise for the reference flow rate.

To conclude, the use of an ordinary peristaltic pump in combination with a tube made of PVC-TOTM is hardly able to satisfy the prescribed requirements in terms of obtainable pressures and/or reduced flow rate performance. In order to compare the characteristics of PVC-TOTM with those of PVC-DOP, some tests have been carried out in which every condition was kept the same with the only exception of the tube material. Specifically, unused tubes with an internal diameter of 8 mm, which ensure an average flow rate of about 320 ml/min, have been subjected to 6 hours uninterrupted operation. As reported above, for tubes made of PVC-DOP, a reduction in flow rate was detected of between 8% and 10%. Conversely, for tubes made of PVC-TOTM, an unacceptable reduction in flow rate was detected of between 20% and 30%.

WO 00/70225 A1 discloses a tubular insert for connecting an extra-corporeal circuit to a peristaltic pump and a peristaltic pump according to the prior art. US 4 558 996 A, GB 1 378 361 A and US 3 737 256 A disclose a peristaltic pump according to the prior art. The object of the present invention is therefore to solve at least partially the problems mentioned in connection with the tubular inserts for extra-corporeal circuits of the known type.

A task of the present invention is to provide an extra-corporeal circuit having a tubular insert which has a high degree of elasticity so as to ensure an optimum performance in terms of flow rate and a limited deterioration thereof during long periods of use. Moreover, the task of the present invention is to provide an extra-corporeal circuit having a tubular insert which has a simple and low-cost design.

Lastly, a task of the present invention is to provide a peristaltic pump suitable for effectively operate with the tubular inserts

The abovementioned object and tasks are achieved by an extra-corporeal circuit according to Claim 1.

The characteristic features and further advantages of the invention will emerge from the description provided hereinbelow, of a number of examples of embodiment, provided by way of a non-limiting example, with reference to the accompanying drawings in which:
Figure 1 shows in schematic form an extra-corporeal circuit used in a therapeutic treatment, specifically a haemodialysis treatment;
Figure 2 schematically shows a tubular insert and peristaltic pump assembly similar to that indicated by II in Figure 1;
Figure 3 schematically shows another tubular insert and peristaltic pump assembly similar to that of Figure 2;
Figure 4 shows a cross-sectional view along the line IV-IV in Figure 3;
Figure 5 shows a front view of the tubular insert according to Figure 2;
Figure 6.a shows a first side view of the tubular insert of Figure 2;

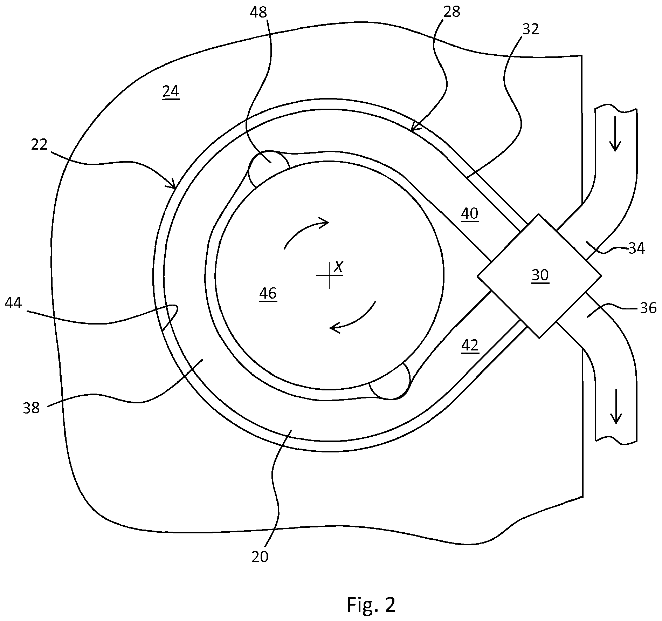

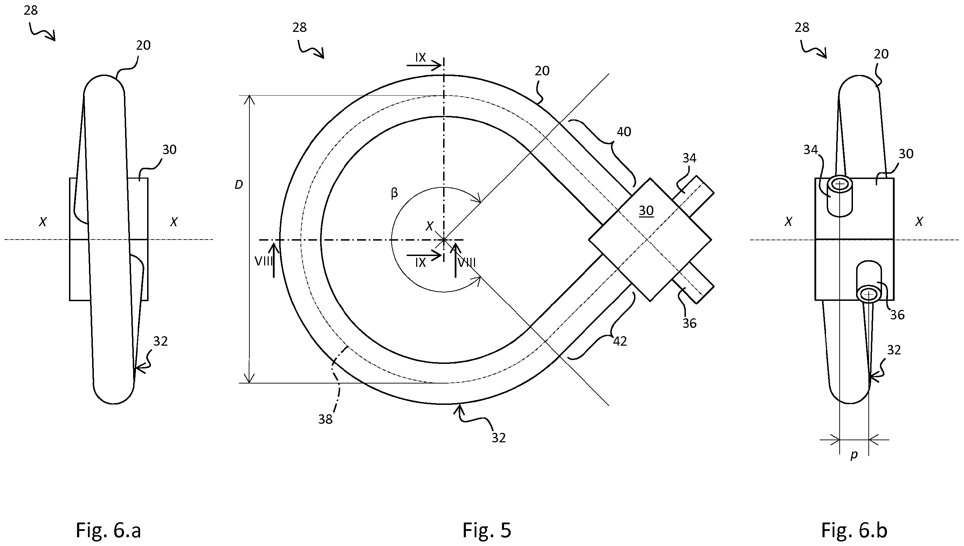

Figure 6.b shows a second side view of the tubular insert of Figure 2;

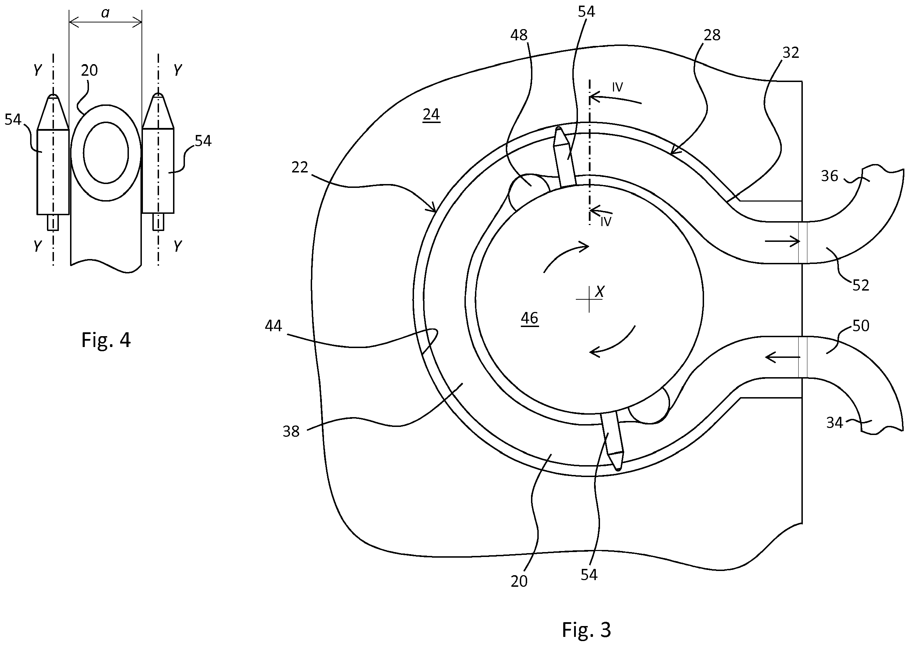

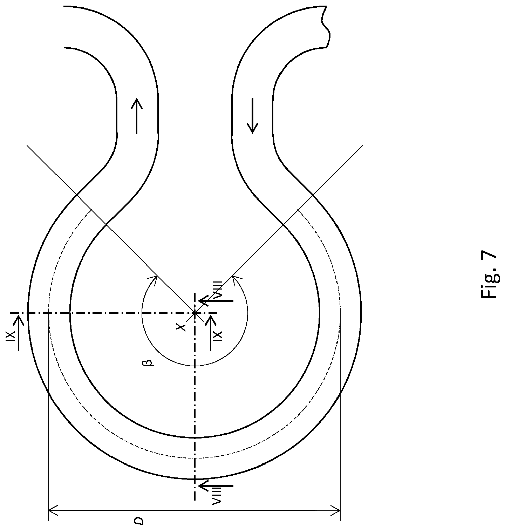

Figure 7 shows a front view of the tubular insert according to Figure 3;

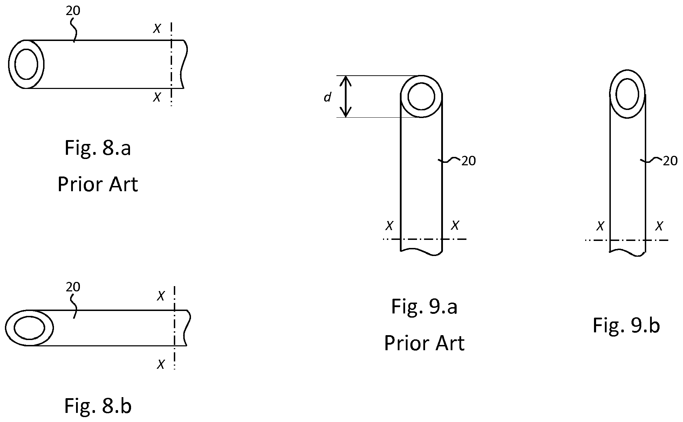

Figure 8.a shows a cross-sectional view along the line VIII-VIII in Figure 5 or 7, in the case of a tubular insert according to the prior art;
Figure 8.b shows a cross-sectional view along the line VIII-VIII in Figure 5 or 7, in the case of the tubular insert according to an example not being part of the invention;
Figure 9.a shows a cross-sectional view along the line IX-IX in Figure 5 or 7, in the case of a tubular insert according to the prior art;
Figure 9.b shows a cross-sectional view along the line IX-IX in Figure 5 or 7, in the case of the tubular insert according to an example not being part of the invention; and

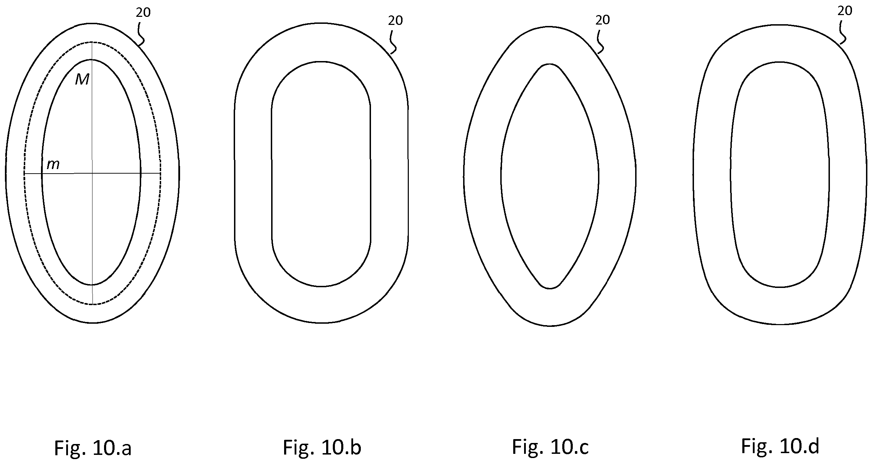

Figures 10 show detailed views of some possible examples of the cross-section according to Figure 9.b.

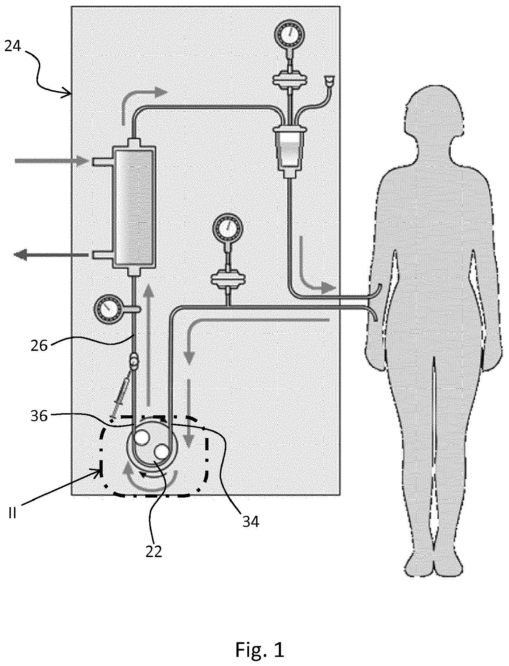

An insert 28 according to the prior art is described below with reference to the accompanying Figures 1, 3 and 7. This tubular insert 28 is indicated below as the "omega insert", due to its overall Ω-shape. Such insert is widely used for connecting the tube 20 with the peristaltic pump 22 when the machine 24 is prepared for a therapeutic treatment, for example a haemodialysis treatment.

According to this known solution, the disposable circuit, denoted overall by 26, comprises the tubular insert 28 shown in detail in Figures 3 and 7. From a theoretic point of view, the omega insert 28 can be simply a length of the tube 20 intended to be introduced in the peristaltic pump 22, rather than a separate element.

As can be seen in the accompanying figures, the tubular insert 28 has an inlet portion 34 and an outlet portion 36. For greater clarity, with specific reference to Figure 1, the inlet portion 34 is that intended to be connected to the circuit section 26 coming from the patient, while the outlet portion 36 is that intended to be connected to the circuit section 26 directed towards the dialysing filter.

The loop 32, as can be seen in the accompanying figures, comprises a broad curve 38 which extends along a circumference having a diameter D. The broad curve 38 is connected to the circuit 26 by means of two tube portions 50, 52. The circumference, along which the curve 38 of the loop 32 extends, defines an axis intended, during use, to coincide with the axis of rotation of the rotor 46 of the peristaltic pump 22. Both these axes are indicated below by a single reference letter X since, during use, they coincide along the same axis.

The curve 38 is the tube portion 20 intended to be inserted between the stator 44 and the rotor 46 of the peristaltic pump 22. In the loop 32 of the tubular insert 28, the curve 38 extends preferably along an arc β having amplitude greater than 180°, so as to be able to effectively co-operate with the rollers 48 of rotor 46, which are generally two in number and arranged at a distance of 180° from each other. For example, in the embodiment of the tubular insert 28 shown in Figure 7, the curve 38 extends along an arc β which has amplitude of 270° or more.

It should be noted that this configuration is a quite theoretical one, and it will be generally assumed by the loop 32 only when it is inserted inside peristaltic pump 22, while, during the non-operative periods where the tubular insert 28 is separated from the peristaltic pump 22, the loop 32 will assume a generally different form determined solely by the reactions inside the tube 20.

It is disclosed a novel type of tubular insert 28 for connecting an extra-corporeal circuit 26 to a peristaltic pump 22. The tubular insert 28 comprises a loop 32 formed by a tube portion 20. The loop 32 comprises a curve 38 which extends around an axis *X*. The tube 20 which forms the curve 38 has an oval cross-section with a major axis M and a minor axis *m* perpendicular to each other, the major axis *M* being longer than the minor axis *m.* In the tubular insert 28 according to the invention, the minor axis *m* of each cross-section of the tube 20 along the curve 38 is parallel to the axis *X*.

In other words, in the omega tubular insert 28, the circumference of the curve 38 lies in the plane of the major axes *M* of the oval cross-sections.

Hereinbelow, the expression "oval" is understood as meaning a general closed and continuous flat curve which bounds a convex region and which has a major axis *M* and a minor axis *m* perpendicular to each other. The oval is a curve preferably without cusps or angled points; this curve has preferably one axis of symmetry, even more preferably has two axes of symmetry. Figures 10 show some closed and continuous curves which represent specific examples of the generic oval shape according to the definition given above. In detail, Figure 10.a. shows an ellipse having major axis *M* and minor axis *m*; Figure 10.b shows a curve composed of two circumference arcs having diameter *m* connected together by two straight segments having length *M*-*m*; Figure 10.c shows a curve composed of two circumference arcs having diameter smaller than *m* connected together by two circumference arcs having diameter greater than *M*; finally, Figure 10.d shows a generic curve inscribed inside a rectangle having sides *M* and *m.* The axes *M* and *m* are indicated solely in Figure 10.a in relation to the mean ellipse within the thickness of the wall. Obviously, the person skilled in the art will have no difficulty in applying the indicated parameters also to other possible cases.

The above described omega insert 28 is preferably used in connection with a specific peristaltic pump 22 which is itself part of the extra-corporeal circuit of the invention. Such peristaltic pump 22 is briefly described hereafter with specific reference to figures 3 and 4 and.

The peristaltic pump of the extra-corporeal circuit according to the invention is suitable for co-operating with a common tube 20 having a circular cross section with an external diameter d (see also figure 9.a). The peristaltic pump 22 comprises, in a manner known per se, a stator 44 and a rotor 46 defining a rotation axis *X* and comprising a plurality of rollers 48 suitable for pressing the tube 20 against the stator 44. In the peristaltic pump 22 of the extra-corporeal circuit according to the invention, the rotor 46 further comprises a couple of radial fingers 54 placed immediately before each roller 48 with respect to the rotation direction; the minimum axial distance *a* between the radial fingers 54 is less than *d*.

In each couple, the two radial fingers 54 are so arranged to contact the tube 20 on opposite sides. More specifically, in each couple, the two radial fingers 54 are so arranged to constrain the tube 20 along an axial direction, i.e. a direction perpendicular to the (radial) direction along which the roller 48 presses the tube 20. Such constraint forces the circular section of a common tube 20 to assume an oval shape according to the invention, i.e. with the minor axis *m* parallel to the axis *X.* Similarly, in case of a tube 20 or insert 28 with an oval cross section, the radial fingers 54 force them to maintain an oval shape according to the invention, i.e. with the minor axis *m* parallel to the axis *X*.

As stated above, the radial fingers 54 are placed before the rollers 48 with respect to the rotation direction. This expression means that, when a tube 20 is engaged with the peristaltic pump 22 and the rotor 46 is rotating, each section of the tube 20 passes first between the two radial fingers 54 and, immediately after that, is pressed by the roller 48 against the stator 44.

Each radial finger 54 preferably comprises a radial roller suitable to rotate around a radial axis *Y* (see figure 4). Such feature permits to minimize the friction in the contact between the radial finger 54 and the wall of the tube 20 during operation of the peristaltic pump 22.

In each radial finger 54, the outer radial end (i.e. the end far from axis *X*) is preferably tapered to a rounded tip (see figures 4 and 5). Such feature entails two advantages. First of all the tapering of the outer end allows, at each turn of the rotor 46, an easy engagement of the portion 50 of the tube 20 between the fingers 54. As can be clearly seen in figure 4, the profile of the two fingers 54 defines a large entrance for the tube 20. Such large entrance easily conducts the portion 50 to the narrow space between the fingers 54 even if the tube 20 is in a slightly improper position. Secondly, the rounded tip minimize the risk for the portion 50 to be pricked or even improperly pressed by the fingers 54.

According to an embodiment of the invention (not shown), the rotor 46 of the peristaltic pump 22 comprises two similar couples of radial fingers 54 for each roller 48: one placed immediately before and the other placed immediately after the roller 48, with respect to the rotation direction.

Another insert 28 according to the prior art is described below with reference to the accompanying Figures 2, 5 and 6. This tubular insert 28 is indicated below as the "alpha insert", due to its overall α-shape. Such particular tubular insert 28 was developed by the Applicant and is widely used for facilitating assembly of the tube 20 with the peristaltic pump 22 when the machine 24 is prepared for a therapeutic treatment, for example a haemodialysis treatment.

According to this known solution, the disposable circuit, denoted overall by 26, comprises the tubular insert 28, commonly called an "alpha clip", shown in detail in Figures 2, 5 and 6.

According to its alpha embodiment, the tubular insert 28 comprises a double connector 30 and a loop 32 formed by a tube portion 20 of suitable length. As can be seen in the accompanying figures, the tubular insert 28 has overall an alpha shape since the inlet portion 34 and the outlet portion 36 overlap each other in the region of the double connector 30. For greater clarity, with specific reference to Figure 1, the inlet portion 34 is that intended to be connected to the circuit section 26 coming from the patient, while the outlet portion 36 is that intended to be connected to the circuit section 26 directed towards the dialysing filter. As can be noted from the accompanying figures 5 and 6, the inlet portion 34 and the outlet portion 36 cross each other, in different planes, inside the double connector 30.

The loop 32, as can be seen in the accompanying figures 2, 5 and 6, comprises a broad curve 38 which extends along a cylindrical helix and is connected to the connector 30 by means of two substantially straight tube segments 40 and 42. The cylindrical helix, along which the curve 38 of the loop 32 extends, defines an axis intended, during use, to coincide with the axis of rotation of the rotor 46 of the peristaltic pump 22. Both these axes are indicated below by a single reference letter *X* since, during use, they coincide along the same axis. Moreover, the cylindrical helix has a pitch *p* which is decidedly smaller than the diameter *D* such that, according to a first approximation, it may be considered that the curve 38 lies in a plane and therefore describes the arc of a circumference. It should be considered, for example, that, according to a first preferred embodiment, the diameter *D* of the cylindrical helix is about 50 mm, while the pitch *p* is only about 6 mm. In view of this approximation, most of the remarks reported above with respect to the omega insert are also valid with respect to the alpha insert.

The approximation of the cylindrical helix portion to an arc of a circumference having the same diameter *D* is further justified in that the peristaltic pump 22 acts on the tubular insert 28 exactly as though the latter were extending in a plane perpendicular to the axis *X* of the rotor 46. This slight geometric discrepancy is fully offset in reality by the deformability of the tube 20.

The curve 38 is the tube portion 20 intended to be inserted between the stator 44 and the rotor 46 of the peristaltic pump 22. In the loop 32 of the tubular insert 28, the curve 38 extends preferably along an arc β having an amplitude greater than 180°, so as to be able to effectively co-operate with the rotor 46, the rollers 48 of which are generally two in number and arranged at a distance of 180° from each other. For example, in the embodiment of the tubular insert 28 shown in Figure 5, the curve 38 extends along an arc β which has an amplitude of about 270°. It should be noted that this configuration is a quite theoretical one, and it will be generally assumed by the loop 32 only when it is inserted inside peristaltic pump 22, while, during the non-operative periods where the tubular insert 28 is separated from the peristaltic pump 22, the loop 32 will assume a generally different form determined solely by the reactions inside the tube 20.

It is also disclosed a novel type of the alpha tubular insert 28. Such tubular insert 28 for connecting an extra-corporeal circuit 26 to a peristaltic pump 22, comprises a double connector 30 and a loop 32 formed by a tube portion 20. The loop 32 comprises a curve 38 which extends around an axis X and is connected to the double connector 30 by means of two substantially straight tube segments 40, 42. The tube 20 which forms the curve 38 has an oval cross-section with a major axis M and a minor axis m perpendicular to each other, the major axis M being longer than the minor axis m. The minor axis m of each cross-section of the tube 20 along the curve 38 is parallel to the axis X.

If the curve described by the loop 32 is approximated to a circumference instead of to a cylindrical helix, in the tubular insert 28 that circumference lies in the plane of the major axes M of the oval cross-sections.

As already explained above, the expression "oval" is understood as meaning a general closed and continuous flat curve which bounds a convex region and which has a major axis *M* and a minor axis *m* perpendicular to each other.

Specific studies conducted by the Applicant have shown that the oval shape of the cross-section of the tube 20 is able to solve most of the problems reported above with respect to the prior art.

As a person skilled in the art is well aware, the mechanical characteristics of the tube 20 depend, among other things, on the intrinsic characteristics of the material and on the geometric characteristics of the involved sections. In the specific case of the tube 20 according to the invention, the geometric characteristics are considerably more advantageous than those of the prior art tubes.

The tube 20 of the tubular insert 28 according to the prior art is generally made of PVC plasticized with a suitable additive, usually DOP. The tube 20 of the tubular insert 28 according to the invention may be made of any material suitable for the specific use. In particular, it is preferably made of PVC plasticized with a suitable additive. The plasticizer can be advantageously TOTM, in order to comply with the new standards which aim to abolish DOP.

With respect to the overall mechanical characteristics of the tube 20, it is to be noted here that the intrinsic characteristics of the PVC material are significantly penalized by the replacement of DOP with TOTM. However, the studies conducted by the Applicant have shown that the geometric characteristics of the oval cross-sections of the tube 20 are able to overcome in an optimum manner this negative consequence.

A number of results of tests carried out by the Applicant, in order to compare the characteristics of the tube 20 with an oval cross-section with those of the conventional tube 20 which has a circular cross-section, where both tubes are made of PVC-TOTM, are described below. A tube 20, the oval section of which has a length equal to the length of the circumference defining the section of the tube of the known type, was considered. A new tube ensures a flow rate of about 320 ml/min and is subject, after 6 hours continuous operation, to a slight reduction in flow rate of between 4% and 5%. From the above it can be understood immediately how the tubular insert ensures a better performance than similar inserts of the known type, both in the case of PVC-TOTM inserts and - this being a particularly significant result - in the case of PVC-DOP inserts. As already reported above, both the omega and the alpha configurations previously described for the loop 32 according to the prior art, are theoretical ones. In practice the tube 20, especially when it is not engaged with the peristaltic pump 22, assumes a different overall configuration which minimizes the deformation energy.

According to its real configuration, the length of the tube 20 intended to originate an omega insert 28 is almost straight. The described omega configuration will be assumed by the loop 32 when it is inserted inside peristaltic pump 22; the act of bending the straight length of tube 20 entails a deformation of the involved cross sections which is disclosed below.

According to its real configuration, the loop 32 of an alpha insert 28 comprises a curve 38 which extends along an arc β having an amplitude smaller than the theoretical one and which has a diameter smaller than the theoretical one. Moreover, the real loop 32 comprises two tube segments 40, 42 which are slightly curved rather than straight. According to both the real alpha and omega configurations, as schematically shown in figures 8.a, the tube 20 has, at the apex of curve 38, an oval cross-section with its major axis *M* parallel to the axis *X,* i.e. opposite to the one described above with respect to the invention. In other words, the tubular insert 28 according to the prior art, spontaneously assume an inefficient configuration, in which the geometric characteristics of the cross-sections noticeably penalize the overall mechanical characteristics of the tube 20.

As the skilled person may easily understand, the radial fingers 54 of the rotor 46 described above are intended to force the tube 20 in the right configuration according to the invention (i.e. with the minor axes *m* of the cross-sections of the tube 20 parallel to axis *X*), even if the tube 20 tends to spontaneously assume the opposite configuration which minimizes the deformation energy (i.e. with the minor axes *m* of the cross-sections of the tube 20 perpendicular to axis *X*). This function of the radial fingers 54 is very important in the case of an omega insert 28 obtained with a length of the tube 20, i.e. an insert 28 in which the tube 20, contrary to the alpha insert, has no constraint with respect to its orientation.

As already described above, the production of the omega tubular inserts 28 according to the prior art simply involves providing a tube portion 20 of suitable length and curving it so as to form the loop 32.

The production of the alpha tubular inserts 28 according to the prior art involves providing a double connector 30 and a tube portion 20 of suitable length which is curved so as to form the loop 32. Each end of the tube 20 is then fitted onto a proper attachment provided on the double connector 30.

In order to produce a tubular insert 28 (either an omega or alpha one) it was proposed using the conventional method described above, subject to the sole condition of using a tube 20 with an oval cross-section instead of the conventional tube 20 with a circular cross-section. Obviously, in order to obtain an insert 28, the tube 20 with an oval cross-section must be curved so that the axis *X* of the loop 32 is parallel to the minor axes *m* of the cross-sections For both the omega insert and, adopting the usual approximation mentioned above, for the alpha insert, it may also be said that the tube 20 must be curved so that the loop 32 remains within the plane which contains the major axes M of the cross-sections of the tube 20 itself.

This method, however, does not produce satisfactory results. It was noted, in fact, that, owing to the geometric characteristics of the oval cross-section, the curvature of the tube 20 in the plane of the major axes *M* does not provide a stable equilibrium for the insert system as a whole. The result is that the tube 20 spontaneously tends to transfer the curvature into a plane containing the minor axes *m*, where the moment of inertia of the oval cross-section is lower. This occurs at least at a distance from the external constraints acting on the tube 20, if any, like the attachments of the double connector 30 in the alpha form. The result is that, in the alpha form with torsional constraints in the region of the attachment points on the double connector 30, the loop 32 no longer lies either along a cylindrical helix or in a plane, but describes a three-dimensional curve which lies well outside the desired plane. Conversely, in the omega form and in the alpha form without torsional constraints acting at the attachments points on the double connector 30, also the ends of the tube 20 tend to rotate. The entire tube 20 thus assumes a position where the curvature of the loop 32 lies in the plane of the minor axes *m*, thus producing a result opposite to that described above for the tubular insert 28 according to the invention.

The method for producing an insert 28 according to an example not being part of the invention comprises the steps of:
- Providing a tube portion 20 which has a circular cross-section and a suitable length;
- Bending the tube portion 20 so as to form the loop 32;
- Subjecting the loop 32 to compression along the axis X so as to ovalize the cross-sections of the tube 20 at least in the zone of the curve 38; and
- At the same time as compression along the axis X, subjecting the loop 32 to a physical treatment suitable to allow internal reorganization of the material which stabilizes its deformed condition.

Accordingly, the loop 32 is formed from a tube portion 20 of the conventional type, typically with a circular cross-section. Only subsequently the loop 32 is subjected to a combined compressive action along the axis X and a physical treatment, e.g. a thermal cycle for heating and cooling the material. In this way it is ensured that the curvature of the loop 32 and the deformation of the cross-sections of the tube 20 caused by compression are rendered permanent by the physical treatment. The physical treatment, e.g. the heating step in the deformed state and the subsequent cooling step, in fact result in internal reorganization of the material which stabilizes its condition. In this way it is ensured that the axis *X* of the loop 32 is stably parallel to the minor axes *m* of the cross-sections or, adopting the usual approximation, that the loop 32 is stably contained within the plane which contains the major axes M of the cross-sections of the tube 20 itself.

According to some examples, the method further comprises the steps of providing a double connector 30 and connecting the loop 32 to the double connector 30.

According to some other examples of the method, ultrasonic treatments and/or radiofrequency treatments are used, instead of the thermal cycle, in order to speed up the production of the tubular insert 28. In order to satisfy specific needs, a combination of thermal, ultrasonic and/or radiofrequency treatments can be used.

As the person skilled in the art may well understood from that described above, the extra-corporeal circuit according to the invention is able to solve at least partly the drawbacks mentioned above in relation to the prior art.

In particular, it will be clear how the tubular insert 28 according to the invention has a high degree of elasticity so as to achieve an optimum performance in terms of flow rate and a limited deterioration thereof following long periods of operation, even in the case where the tube is made of PVC-TOTM.

Moreover, the peristaltic pump according to the invention is able to obtain, from the well-known tubular inserts, advantages similar to those obtainable with the tubular inserts 28 according to the invention. The pump according to the invention is suitable for co-operating, without distinction, with the insert 28 according to the invention (both in its alpha and omega form) or with a tubular insert commonly used in the peristaltic pumps according to the prior art.

Lastly, it will also be clear to the person skilled in the art how both the tubular inserts 28, the peristaltic pump 22 and the method for producing the insert 28 constitute a simple and low-cost solution.

With regard to the embodiments of the tubular insert, of the peristaltic pump and of the production method described above, the person skilled in the art may, in order to satisfy specific requirements, make modifications to and/or replace elements described with equivalent elements, without thereby departing from the scope of the accompanying claims.

## Claims

1. Extra-corporeal circuit (26) for circulation of a physiological liquid, comprising a tube (20) having a circular cross-section with an external diameter d and a peristaltic pump (22) suitable for co-operating with the tube (20),
wherein the peristaltic pump (22) comprises a stator (44) and a rotor (46) defining a rotation axis *X*, wherein the tube comprises a loop formed by a tube portion, the loop comprising a curve (38) extending around the axis *X*, and the rotor comprising a plurality of rollers (48) suitable for pressing the tube (20) against the stator (44), the rotor (46) further comprising a couple of radial fingers (54) placed immediately before each roller (48) with respect to the rotation direction,
the extra-corporeal circuit (26) being **characterized in that** a minimum axial distance (*a*) between the radial fingers (54) is less than *d* so that the radial fingers (54) force the tube (20) to assume an oval cross-section with a major axis M and a minor axis *m* perpendicular to each other, the major axis *M* being longer than the minor axis *m,* and wherein the minor axis *m* of each cross-section of the tube (20) along the curve (38) is parallel to the axis *X*.

2. Extra-corporeal circuit (26) according to claim 1, wherein each radial finger (54) of the peristaltic pump (22) comprises a radial roller suitable to rotate around a radial axis Y so as to minimize the friction in the contact between the radial finger (54) and the wall of the tube (20) during operation of the peristaltic pump (22).

3. Extra-corporeal circuit (26) according to claim 1 or 2, wherein the outer radial end of each radial finger (54) is tapered to a rounded tip.

4. Extra-corporeal circuit (26) according to any one of claims 1 to 3, wherein the rotor (46) of the peristaltic pump (22) comprises two couples of radial fingers (54) for each roller (48), one placed immediately before and the other placed immediately after the roller (48), with respect to the rotation direction.

5. Machine (24) suitable for carrying out therapeutic treatments, comprising an extra-corporeal circuit according to any claim 1 to 4.

## Patentansprüche

1. Außerkörperlicher Kreislauf (26) für die Zirkulation einer physiologischen Flüssigkeit, umfassend einen Schlauch (20) mit einem kreisförmigen Querschnitt mit einem Außendurchmesser d und eine Peristaltikpumpe (22), die geeignet ist, mit dem Schlauch (20) zusammenzuwirken,
wobei die Peristaltikpumpe (22) einen Stator (44) und einen Rotor (46) umfasst, die eine Rotationsachse X definieren,
wobei der Schlauch eine durch einen Schlauchabschnitt gebildete Schleife umfasst,
wobei die Schleife eine Kurve (38) aufweist, die sich um die Achse X herum erstreckt, und
der Rotor eine Mehrzahl von Walzen (48) aufweist, die geeignet sind, den Schlauch (20) gegen den Stator (44) zu drücken,
wobei der Rotor (46) ferner radiale Finger (54) aufweist, die in Bezug auf die Drehrichtung unmittelbar vor jeder Walze (48) angeordnet sind,
wobei der außerkörperliche Kreislauf (26) **dadurch gekennzeichnet ist, dass** ein minimaler axialer Abstand (a) zwischen den radialen Fingern (54) kleiner als d ist, so dass die radialen Finger (54) den Schlauch (20) zwingen, einen ovalen Querschnitt mit einer größeren Achse M und einer kleineren Achse m anzunehmen, die senkrecht zueinander sind,
wobei die größere Achse M länger als die kleinere Achse m ist, und
wobei die kleinere Achse m jedes Querschnitts des Schlauchs (20) entlang der Kurve (38) parallel zu der Achse X ist.

2. Außerkörperlicher Kreislauf (26) gemäß Anspruch 1, wobei jeder radiale Finger (54) der Peristaltikpumpe (22) eine radiale Walze aufweist, die geeignet ist, sich um eine radiale Achse Y zu drehen, um so die Reibung im Kontakt zwischen dem radialen Finger (54) und der Wand des Schlauchs (20) während des Betriebs der Peristaltikpumpe (22) zu minimieren.

3. Außerkörperlicher Kreislauf (26) gemäß Anspruch 1 oder 2, wobei das äußere radiale Ende jedes radialen Fingers (54) zu einer abgerundeten Spitze verjüngt ist.

4. Außerkörperlicher Kreislauf (26) gemäß einem der Ansprüche 1 bis 3, wobei der Rotor (46) der Peristaltikpumpe (22) zwei Paare von radialen Fingern (54) für jede Walze (48) aufweist, von denen das eine in Bezug auf die Drehrichtung unmittelbar vor und das andere unmittelbar nach der Walze (48) angeordnet ist.

5. Maschine (24), die zur Durchführung von therapeutischen Behandlungen geeignet ist und einen außerkörperlichen Kreislauf gemäß einem der Ansprüche 1 bis 4 aufweist.

## Revendications

1. Circuit extracorporel (26) pour la circulation d'un liquide physiologique comprenant un tube (20) ayant une section transversale circulaire avec un diamètre externe d et une pompe péristaltique (22) appropriée pour coopérer avec le tube (20),
dans lequel la pompe péristaltique (22) comprend un stator (44) et un rotor (46) définissant un axe de rotation X, dans lequel le tube comprend une boucle formée par une partie de tube, la boucle comprenant une courbe (38) s'étendant autour de l'axe X, et le rotor comprenant une pluralité de rouleaux (48) appropriés pour comprimer le tube (20) contre le stator (44), le rotor (46) comprenant en outre une paire de doigts radiaux (54) placés immédiatement avant chaque rouleau (48) par rapport à la direction de rotation,
le circuit extracorporel (26) étant **caractérisé en ce qu'**une distance axiale minimum (a) entre les doigts radiaux (54) est inférieure à d de sorte que les doigts radiaux (54) forcent le tube (20) à adopter une section transversale ovale avec un axe majeur M et un axe mineur m perpendiculaire entre eux, l'axe majeur M étant plus long que l'axe mineur m, et dans lequel l'axe mineur m de chaque section transversale du tube (20) le long de la courbe (38) est parallèle à l'axe X.

2. Circuit extracorporel (26) selon la revendication 1, dans lequel chaque doigt radial (54) de la pompe péristaltique (22) comprend un rouleau radial approprié pour tourner autour d'un axe radial Y afin de minimiser la friction dans le contact entre le doigt radial (54) et la paroi du tube (20) pendant le fonctionnement de la pompe péristaltique (22).

3. Circuit extracorporel (26) selon la revendication 1 ou 2, dans lequel l'extrémité radiale externe de chaque doigt radial (54) est progressivement rétrécie vers une pointe arrondie.

4. Circuit extracorporel (26) selon l'une quelconque des revendications 1 à 3, dans lequel le rotor (46) de la pompe péristaltique (22) comprend deux paires de doigts radiaux (54) pour chaque rouleau (48), un placé immédiatement avant et l'autre placé immédiatement après le rouleau (48), par rapport à la direction de rotation.

5. Machine (24) appropriée pour réaliser des traitements thérapeutiques, comprenant un circuit extracorporel selon l'une quelconque des revendications 1 à 4.
